(19) Europäisches Patentamt
European Patent Office
Office européen des brevets

(11) **EP 3 240 923 B1**

(12) **EUROPEAN PATENT SPECIFICATION**

(45) Date of publication and mention
of the grant of the patent:
**06.03.2019 Bulletin 2019/10**

(21) Application number: **15816599.3**

(22) Date of filing: **02.12.2015**

(51) Int Cl.:
*D04H 1/587* (2012.01)        *D04H 1/64* (2012.01)

(86) International application number:
**PCT/US2015/063314**

(87) International publication number:
**WO 2016/109086 (07.07.2016 Gazette 2016/27)**

(54) **LOTION FOR NONWOVEN BINDER**

LOTION FÜR VLIESBINDER

LOTION POUR LIANT DE NON-TISSÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.12.2014 US 201462097840 P**

(43) Date of publication of application:
**08.11.2017 Bulletin 2017/45**

(73) Proprietor: **Rohm and Haas Company
Philadelphia, PA 19106 (US)**

(72) Inventors:
• **NUNN, Maureen B.**
  **Lower Gwynedd, PA 19002 (US)**
• **KLINE, Debra A.**
  **Collegeville, PA 19426 (US)**
• **RICE, Katherine Sue**
  **Glenside, PA 19038 (US)**

(74) Representative: **Boult Wade Tennant LLP
Verulam Gardens
70 Gray's Inn Road
London WC1X 8BT (GB)**

(56) References cited:
**EP-A2- 2 447 306     WO-A1-2013/154879**

## Description

### Field of Invention

The instant invention relates to a method for forming dispersible nonwoven substrate compositions and the dispersible nonwoven substrates made therefrom.

### Background of the Invention

Currently, the wastewater treatment industry is focused on disposable products, such as wet wipes, which cause clogging in equipment causing difficulties and leading to downtime at wastewater treatment facilities. Therefore, wipes having adequate tensile strength while in use, but that are easily dispersible in water are desirable.

Various binders have been developed to increase dispersibility in water after toilet flushing. Examples include binders which take advantage of the differences in pH between the liquid/lotion used in the wipe and water to provide dispersibility. This way, a wipe can be intact while being used and disintegrate once being placed into the wastewater system. In order for this to happen, the pH of the lotion portion of the wipe must be kept between 4 and 5.5. However, sometimes the pH of the lotion portion can shift after a certain amount of time. If the pH shifts to above 5-5.5, then the strength of the wipe is lost.

Therefore, lotion formulations which can maintain a pH of between 4 and 5.5 after being added to a dispersible wipe are desirable.

WO 2013/154879 relates to a method for forming a dispersible nonwoven.

### Summary of the Invention

The instant invention provides a method for forming a dispersible nonwoven substrate in an aqueous medium. The instant invention also provides baby wipes, personal care wipes, cleaning wipes, or any wet nonwoven having a lotion of below pH 5.5 made from the dispersible nonwoven substrate formed by this method.

In one embodiment of the invention, there is provided a method for forming a dispersible nonwoven substrate comprising the steps of: a) emulsion polymerizing at least one mono-ethylenically unsaturated monomer to form an emulsion polymer; b) neutralizing the emulsion polymer with a neutralizer selected from the group consisting of an amine having a pKb of 4 to7, an alkali hydroxide, and combinations thereof to form the aqueous nonwoven binder; c) contacting a nonwoven substrate with said aqueous nonwoven binder to form a contacted nonwoven substrate;

d) heating the contacted nonwoven substrate to a temperature of from 120°C to 220°C to form a heated contacted nonwoven substrate; and e) immersing the contacted heated nonwoven substrate in an aqueous medium having a final pH of less than 5.5 to provide a dispersible nonwoven substrate wherein the aqueous medium contains a buffer comprising a mixture of citric acid and sodium citrate.

In alternative, the instant invention provides a dispersible nonwoven substrate formed by the method of any of the preceding embodiment.

In an alternative embodiment, the instant invention provides dispersible wipes, such as baby wipes, personal care wipes, cleaning wipes, or any wet nonwoven containing a lotion having a pH of below 5.5 in accordance with any of the preceding embodiments.

In an alternative embodiment, the instant invention provides a dispersible nonwoven substrate comprising: a) a fibrous substrate; and b) a nonwoven binder comprising an emulsion polymer having a polydispersity index of at least 10, a weight average molecular weight of less than 1,000,000 and from 8 weight percent to 22 weight percent acid monomer c) a lotion having a pH in the range of from 4 to 5.5, which also comprises a buffer comprising a mixture of citric acid and sodium citrate.

### Detailed Description of the Invention

The instant invention provides a method for forming a dispersible nonwoven substrate in an aqueous medium and wipes such as baby, personal care, or cleaning wipes, or any wet nonwoven whose lotion is below pH 5.5 made from the dispersible nonwoven substrate formed by this method.

In the method for forming a dispersible nonwoven substrate in an aqueous medium of the present invention by "aqueous" herein is meant a composition in which the continuous phase is water or, in the alternative, a mixture including predominantly water but also optionally including water-miscible solvent, biocides, emoliants, buffers, chelants, and surfactants and other ingredients. By "dispersible" herein is meant that the nonwoven substrate can, under appropriate conditions, be caused to disintegrate into at least one of: smaller pieces, aggregates of fibers, individual fibers, and mixtures thereof.

By "nonwoven" herein is meant a fabric-like assembly of fibers typically in sheet or web form that is not a woven or knitted material. The nonwoven substrate includes paper; nonwoven fabrics; felts and mats; or other assemblies of fibers. The nonwoven substrate may include: cellulosic fibers such as cotton, rayon, and wood pulp; synthetic fibers such as polyester, glass, and nylon; bicomponent fibers; and mixtures thereof. In an embodiment, the nonwoven substrate has a predominant amount of fiber capable of engaging in hydrogen-bonding. In an alternative embodiment, the nonwoven substrate includes a predominant amount of cellulosic fiber. The nonwoven substrate may be formed by methods known in the art such as, for example, wet-laid, air-laid, spunbonding, spunmelt, and hydroentangling web formation. The fibers are typically selected so as their length and composition is not inimical to the ultimate dispersibility of the treated nonwoven substrate.

The method for forming a dispersible nonwoven substrate comprises the steps of: a) emulsion polymerizing at least one mono-ethylenically unsaturated monomer to form an emulsion polymer; b) neutralizing the emulsion polymer with a neutralizer selected from the group consisting of an amine having a pKb of 4 to7, an alkali hydroxide, and combinations thereof to form the aqueous nonwoven binder; c) contacting a nonwoven substrate with said aqueous nonwoven binder to form a contacted nonwoven substrate; d) heating the contacted nonwoven substrate to a temperature of from 120°C to 220°C to form a heated contacted nonwoven substrate; and e) immersing the contacted heated nonwoven substrate in an aqueous medium having a final pH of less than 5.5 to provide a dispersible nonwoven substrate wherein the aqueous medium contains a buffer comprising a mixture of citric acid and sodium citrate.

The dispersible nonwoven substrate includes an emulsion polymer; that is, a polymer prepared by the free radical polymerization of ethylenically-unsaturated monomers in an aqueous emulsion polymerization process.

In various embodiments, the emulsion polymer can include, as copolymerized units, from 8 to 22 weight percent acid monomers, based on the weight of the emulsion polymer. All ranges between 8 and 22 weight percent are included herein and disclosed herein; for example, the weight percent of acid monomers can be from a lower limit of 8, 12.5, or 14 to an upper limit of 11, 16, 20, or 22.

The emulsion polymer has a polydispersity index of at least 10. Any and all ranges greater than or equal to 10 are included herein and disclosed herein, for example, the emulsion polymer can have a polydispersity index of 15, 20, 30, 40, or 50.

The emulsion polymer also has a weight average molecular weight of less than 1,000,000. Any and all ranges less than 1,000,000 are included herein and disclosed herein, for example, the emulsion polymer can have a weight average molecular weight of less than 900,000, less than 500,000, or less than 400,000.

In various embodiments, particular ranges of acid monomer weight percent are combined with particular weight average molecular weight ranges in order to obtain the desired viscosity to produce a binder material having a solids content of at least 20 weight percent after neutralization. A composition having at least 20 weight percent solids is generally required for commercial shipping. In an embodiment, the emulsion polymer has a weight average molecular weight of less than 500,000 and from 12.5 weight percent to 20 weight percent acid monomer. In another embodiment, the emulsion polymer has a weight average molecular weight of less than 400,000 and from 14 weight percent to 16 weight percent acid monomer. In yet another embodiment, the emulsion polymer has a weight average molecular weight of less than 900,000 and from 8 weight percent to 11 weight percent acid monomer.

Acid monomers can include monoacid monomers and diacid monomers. Monoacid monomers include, for example, carboxylic acid monomers such as, for example, acrylic acid, methacrylic acid, crotonic acid, monomethyl itaconate, monomethyl fumarate, monobutyl fumarate. Examples of diacid monomers include, but are not limited to itaconic acid, fumaric acid, maleic acid; including their anhydrides, salts, and mixtures thereof.

The emulsion polymer includes at least one other copolymerized ethylenically unsaturated monomer such as, for example, a (meth)acrylic ester monomer including methyl (meth)acrylate, ethyl (meth)acrylate, butyl (meth)acrylate, 2-ethylhexyl (meth)acrylate, decyl (meth)acrylate, hydroxyethyl (meth)acrylate, hydroxypropyl (meth)acrylate, ureido-functional (meth)acrylates and acetoacetates, acetamides or cyanoacetates of (meth)acrylic acid; styrene or substituted styrenes; vinyl toluene; butadiene; vinyl acetate or other vinyl esters; vinyl monomers such as vinyl chloride, vinylidene chloride; and (meth)acrylonitrile. The use of the term "(meth)" followed by another term such as (meth)acrylate or (meth)acrylamide as used throughout the disclosure, refers to both acrylates and methacrylates or acrylamides and methacrylamides, respectively. The at least one other copolymerized ethylenically unsaturated monomer is selected so that the emulsion polymer will have a Tg within the required range. In an embodiment, the emulsion polymer comprises ethyl acrylate, styrene, acrylic acid, and itaconic acid.

Mixtures of emulsion polymers having different compositions are also contemplated. For a mixture of two or more emulsion polymers, the acid monomer content and the Tg shall be determined from the overall composition of the emulsion polymers without regard for the number or individual composition of the emulsion polymers therein.

The emulsion polymerization techniques used to prepare the emulsion polymer are well known in the art such as, for example, as disclosed in U.S. Patents No. 4,325,856; 4,654,397; and 4,814,373. In various embodiments, the emulsion polymerization is performed at a reaction temperature of from room temperature to 100°C, depending on the initiation process (eg., thermal or redox). Conventional surfactants may be used such as, for example, anionic and/or nonionic emulsifiers such as, for example, alkali metal or ammonium alkyl sulfates, alkyl sulfonic acids, fatty acids, copolymerizable surfactants, and oxyethylated alkyl phenols. Preferred are anionic emulsifiers. The amount of surfactant used is usually 0.1% to 6% by weight, based on the weight of total monomer. Either thermal or redox initiation processes may be used. Conventional free radical initiators may be used such as, for example, hydrogen peroxide, t-butyl hydroperoxide, t-amyl hydroperoxide, ammonium and/or alkali persulfates, and water-soluble azo compounds such as azobis cyanovaleric acid, typically at a level of 0.01% to 3.0% by weight, based on the weight of total monomer. In an embodiment, the initiator is present in a range of from 0.5% to 1.5% by weight, based on the weight of total monomer. Redox systems using the same initiators coupled with a suitable reductant such as, for example, sodium sulfoxylate formaldehyde, sodium hydrosulfite, isoascorbic acid, hydroxylamine sulfate and sodium bisulfite may be used at similar levels, optionally in combination with metal ions such as, for example iron and copper, optionally further including complexing agents for the metal. Chain transfer agents such as mercaptans may be used to lower the molecular weight of the polymers. The monomer mixture may be added neat or as an emulsion in water. The monomer mixture may be added in a single addition or in multiple additions or continuously over the reaction period using a uniform or varying composition. Additional ingredients such as, for example, free radical initiators, oxidants, reducing agents, chain transfer agents, neutralizers, surfactants, and dispersants may be added prior to, during, or subsequent to any of the stages. In various embodiments, the emulsion polymer may be prepared by a multistage emulsion polymerization process, in which at least two stages differing in composition are polymerized in sequential fashion. The molecular weight of the final polymer falls within the above molecular weight ranges.

In an embodiment of the present invention, the emulsion polymer is neutralized with a neutralizer selected from the group consisting of an amine with a pKb of 4 to 7, an alkali hydroxide, and combinations thereof. Examples of alkali hydroxides that can be used as neutralizers include, but are not limited to sodium hydroxide and potassium hydroxide. Examples of amines having a pKb of 4 to 7 include, but are not limited to tromethamine, monoethanol amine, diethanol amine, and triethanol amine. In various embodiments, the neutralizer can contain from 0 to 100 weight % amine. Any and all ranges between 0 and 100 weight % are included herein and disclosed herein, for example, the neutralizer can contain 40, 50, 60, or 75 weight % amine. In various embodiments, the neutralizer can contain from 0 to 100 weight % alkali hydroxide. Any and all ranges between 0 and 100 weight % are included herein and disclosed herein, for example, the neutralizer can contain 40, 50, 60, or 75 weight % alkali hydroxide.

The calculated glass transition temperature ("Tg") of the emulsion polymer, before neutralization, is generally from -20°C to 30°C. Tgs of the polymers herein are those calculated using the Fox equation (T.G. Fox, Bull. Am. Physics Soc., Volume 1, Issue No. 3, p. 123(1956)). That is, for example, for calculating the Tg of a copolymer of monomers M1 and M2,

$$1/Tg(calc.) = w(M1)/Tg(M1) + w(M2)/Tg(M2)$$

,

wherein

Tg(calc.) is the glass transition temperature calculated for the copolymer
w(M1) is the weight fraction of monomer M1 in the copolymer
w(M2) is the weight fraction of monomer M2 in the copolymer
Tg(M1) is the glass transition temperature of the homopolymer of M1
Tg(M2) is the glass transition temperature of the homopolymer of M2,

all temperatures being in °K.

The glass transition temperature of homopolymers may be found, for example, in "Polymer Handbook", edited by J. Brandrup and E.H. Immergut, Interscience Publishers. In any event the following homopolymer Tgs are to be used in the Fox equation calculation for the following polyacids: poly(methacrylic acid) Tg=185 °C; poly(acrylic acid) Tg=106 °C; poly(itaconic acid) Tg=154 °C; and poly(maleic anhydride) Tg=154 °C.

The average particle diameter of the emulsion polymer particles is typically from 30 nanometers to 500 nanometers, preferably from 200 nanometers to 400 nanometers as measured by a Brookhaven Model BI-90 Particle Sizer supplied by Brookhaven Instrument Corp., Holtsville, NY.

The aqueous nonwoven binder may include, in addition to the emulsion polymer, conventional treatment components such as, for example, emulsifiers, pigments, fillers or extenders, anti-migration aids, curing agents, coalescents, surfactants, biocides, plasticizers, organosilanes, anti-foaming agents, corrosion inhibitors, colorants, waxes, other polymers, and anti-oxidants.

In the method for forming a dispersible nonwoven substrate in an aqueous medium of the present invention the nonwoven substrate is contacted with the aqueous nonwoven binder. Typically the ratio of nonwoven binder to that of the contacted nonwoven substrate on a dry weight basis expressed as a percentage, also known as % add-on, is from 1% to 25%, preferably from 1% to 10%, selected depending on the strength of the nonwoven substrate and the desired end use. The nonwoven substrate is contacted with the aqueous nonwoven binder using conventional application techniques such as, for example, air or airless spraying, padding, saturating, roll coating, curtain coating, gravure printing, and the like. The nonwoven substrate may be contacted with the aqueous nonwoven binder so as to provide binder at or near one or both surfaces or distributed uniformly, or not, throughout the structure. It is also contemplated that the aqueous nonwoven binder may be applied in a nonuniform manner to one or both surfaces when a patterned distribution is desired.

In the method for forming a dispersible nonwoven substrate in an aqueous medium of the present invention the nonwoven substrate that has been contacted with the aqueous nonwoven binder is heated to a temperature of from 120 °C to 220 °C, preferably from 140 °C to 180 °C, for a time sufficient to achieve an acceptable level of dryness. In an embodiment, the composition is heated at a temperature and for a time sufficient to substantially dry but not to substantially cure the composition.

In the method for forming a dispersible nonwoven substrate in an aqueous medium of the present invention the contacted heated nonwoven substrate is immersed in an aqueous medium having a final pH<5.5, a final pH of from 3.0 to 4.99 in various embodiments, to provide a dispersible nonwoven in an aqueous medium. By "final pH" herein is meant the pH (measured at 20°C) of the aqueous medium in which the contacted heated nonwoven is immersed. The required pH of the aqueous medium is believed to contribute to a beneficial level of wet strength to the heated treated nonwoven and is also a suitable pH for certain compositions such as, for example, wipe solutions and lotions, in which the heated treated nonwoven may be stored. In an embodiment, the aqueous medium is a lotion. Typically the weight of aqueous medium is from 0.1 to 10, preferably from 0.5 to 5 times the weight of the contacted heated nonwoven substrate.

If the final pH<5.5 is not achieved with the selected nonwoven and aqueous medium and the amounts thereof, the pH is adjusted to the desired range by the addition of a buffer of acidic material prior to, during, or after the immersing step. In an embodiment of the present invention, the acidic material is citric acid. In another embodiment, the acidic material is a combination of citric acid and sodium citrate. In various embodiments, between 1 and 20 weight percent of buffer, based on the total weight of the aqueous medium, is needed to adequately adjust the pH. The exact amount depends on the amount of the binder polymer and the specific buffer used. Less buffer can be used if the non-woven substrate is pre-neutralized with an acid. In an embodiment, the non-woven substrate can be pre-neutralized with a 3 % solution of citric acid.

The dispersible nonwoven of the present invention can then be immersed in an excess of an aqueous medium at a pH of >6, preferably at a final pH of >6.5; preferably at a final pH of from 6.8 to 10.0. By "an excess of an aqueous medium" is meant herein that the weight of the aqueous medium is greater than the weight of the dispersible nonwoven. The wet strength of the heated treated nonwoven is sufficiently reduced at the designated pH (measured at 20°C) so as to facilitate its disintegration into at least one of: smaller pieces, aggregates of fibers, individual fibers, and mixtures thereof. Of course any mechanical forces that may be applied will aid in this dispersibility process, such as, for example, if the treated nonwoven were deposited in an excess of water at a pH of >6 and subjected to a shear force such as in a toilet flushing action.

Embodiments of the present invention can be used to make a variety of wipes, such as baby, personal care, and cleaning wipes. Other wipes having a pH of below about 5 or 6 can also be made using embodiments of the present invention.

## Examples

**Substrate:** Whatman4 Filter Paper

Polymer was applied using a Birch Bros. padder at speed 5 and 35# psi. Samples prepared are described in Table I with the percent dry polymer add-on listed.

## Lotion Modification

Parents Choice lotion was extracted from a wipe using a Birch Bros. padder at speed 5 and 40# psi. A portion of the extracted lotion was used as extracted and a portion modified with the addition of fifty mmolar sodium citrate/citric acid buffer..

## Sample Preparation

Ten 1 inch by 4 inch strips of Whatman 4 filter paper were cut and weighed for each week of testing. Lotion was added until the weight was 3-4 times the original weight of the wipe. The strips were wrapped in aluminum foil and placed in a 50°C oven. Samples were tested after 2, 3, and 4 weeks. The samples that were prepared are shown in Table 1.

Table 1 - Initial Properties

| Wt % Dry Polymer | Dry Tensile g/in. | STDEV | Lotion Tensile g/in. | STDEV | pH 7 Tensile g/in. | STDEV | % Dispersed pH 6 |
|---|---|---|---|---|---|---|---|
| 0 | 3251.88 | 282.898 | 163.37 | 16.261 | 130.17 | 36.567 | 100.00 |
| 5% | 5644.66 | 515.918 | 678.03 | 69.152 | 321.74 | 28.898 | 100.00 |
| 15% | 9295.95 | 633.113 | 797.6379 | 122.1067 | 265.56 | 49.19 | 73.88 |

The initial tensile strength and the tensile strength after 2, 3, and 4 weeks are shown in Table 2.

Table 2- Tensile Strength

| | 0% Buffer | STDEV | 50 mmolar Citric Acid/ Sodium Citrate Buffer | STDEV |
|---|---|---|---|---|
| INITIAL | 154.497 | 34.8058 | 797.6379 | 122.1067 |
| 2 WEEKS | 162.665 | 16.5327 | 719.508 | 66.2511 |
| 3 WEEKS | 155.664 | 18.2936 | 914.924 | 85.3587 |
| 4 WEEKS | 194.716 | 15.4243 | 924.026 | 145.0686 |

The addition of 50 mmolar citric acid/sodium citrate buffer to the baby wipe lotion can be used to maintain the strength of the wet wipe for 4 weeks at 50°C. This is equivalent to one year on the shelf. With no modification, the Tensile Strength loss was 50% in 7 days at 40°C.

**Tensile Strength Test Method**

Tensile measurements were performed using an EJA Vantage Materials Tester 24" from Thwing- Albert.

- Speed 12" min.
- Jaws 2" Gap

**Claims**

1. A method for forming a dispersible nonwoven substrate comprising the steps of:

   a) emulsion polymerizing at least one mono-ethylenically unsaturated monomer to form an emulsion polymer; and
   b) neutralizing the emulsion polymer with a neutralizer selected from the group consisting of an amine having a pKb of 4 to 7, an alkali hydroxide, and combinations thereof to form the aqueous nonwoven binder;
   c) contacting a nonwoven substrate with said aqueous nonwoven binder to form a contacted nonwoven substrate;
   d) heating the contacted nonwoven substrate to a temperature of from 120°C to 220°C to form a heated contacted nonwoven substrate; and
   e) immersing the contacted heated nonwoven substrate in an aqueous medium having a final pH of less than 5.5 to provide a dispersible nonwoven substrate wherein the aqueous medium contains a buffer comprising a mixture of citric acid and sodium citrate.

2. A method in accordance with claim 1 wherein the emulsion polymer comprises ethyl acrylate, styrene, acrylic acid, and itaconic acid.

3. A method in accordance with claim 1 wherein the emulsion polymer has a weight average molecular weight of less than 1,000,000.

4. A method in accordance with claim 1 wherein the emulsion polymer has a polydispersity index of at least 10.

**5.** A method in accordance with claim 1 wherein the heated contacted nonwoven substrate is neutralized with a composition comprising an acid before being immersed in the aqueous medium in step e).

**6.** A method in accordance with claim 1 wherein the neutralization step results in a polymer that is 80 % to 100 % neutralized.

**7.** A method in accordance with claim 1 wherein the emulsion polymer, before neutralization in step b), has a glass transition temperature in the range of from -20°C to 30°C.

**8.** A dispersible nonwoven substrate comprising:

a) a fibrous substrate; and
b) a nonwoven binder comprising an emulsion polymer having a polydispersity index of at least 10, a weight average molecular weight of less than 1,000,000 and from 8 weight percent to 22 weight percent acid monomer
c) a lotion having a pH in the range of from 4 to 5.5, which also comprises a buffer comprising a mixture of citric acid and sodium citrate.

**9.** A dispersible wipe product prepared by the method of claim 1.

**10.** A baby wipe prepared with the dispersible wipe product of claim 9.

**11.** A personal care wipe prepared with the dispersible wipe product of claim 9.

**12.** A cleaning wipe prepared with the dispersible wipe product of claim 9.

**13.** A disinfecting wipe prepared with the dispersible wipe product of claim 9.

**14.** An industrial wipe prepared with the dispersible wipe product of claim 9.

**Patentansprüche**

**1.** Ein Verfahren zum Bilden eines dispergierbaren Vliesstoffsubstrats, das die folgenden Schritte beinhaltet:

a) Emulsionspolymerisieren mindestens eines monoethylenisch ungesättigten Monomers, um ein Emulsionspolymer zu bilden; und
b) Neutralisieren des Emulsionspolymers mit einem Neutralisationsmittel, ausgewählt aus der Gruppe, bestehend aus einem Amin mit einem pKb-Wert von 4 bis 7, einem Alkalihydroxid und Kombinationen davon, um das wässrige Vliesstoffbindemittel zu bilden;
c) In-Kontakt-Bringen eines Vliesstoffsubstrats mit dem wässrigen Vliesstoffbindemittel, um ein in Kontakt gebrachtes Vliesstoffsubstrat zu bilden;
d) Erwärmen des in Kontakt gebrachten Vliesstoffsubstrats auf eine Temperatur von 120 °C bis 220 °C, um ein erwärmtes, in Kontakt gebrachtes Vliesstoffsubstrat zu bilden; und
e) Eintauchen des in Kontakt gebrachten, erwärmten Vliesstoffsubstrats in ein wässriges Medium mit einem End-pH-Wert von weniger als 5,5, um ein dispergierbares Vliesstoffsubstrat bereitzustellen, wobei das wässrige Medium einen Puffer enthält, der eine Mischung von Zitronensäure und Natriumcitrat beinhaltet.

**2.** Verfahren gemäß Anspruch 1, wobei das Emulsionspolymer Ethylacrylat, Styrol, Acrylsäure und Itaconsäure beinhaltet.

**3.** Verfahren gemäß Anspruch 1, wobei das Emulsionspolymer ein Molekulargewicht im Gewichtsmittel von weniger als 1 000 000 aufweist.

**4.** Verfahren gemäß Anspruch 1, wobei das Emulsionspolymer einen Polydispersitätsindex von mindestens 10 aufweist.

**5.** Verfahren gemäß Anspruch 1, wobei das erwärmte, in Kontakt gebrachte Vliesstoffsubstrat vor dem Eintauchen in das wässrige Medium in Schritt e) mit einer Zusammensetzung, die eine Säure beinhaltet, neutralisiert wird.

**6.** Verfahren gemäß Anspruch 1, wobei der Neutralisierungsschritt ein Polymer ergibt, das zu 80 % bis 100 % neutralisiert ist.

**7.** Verfahren gemäß Anspruch 1, wobei das Emulsionspolymer vor dem Neutralisierungsschritt in Schritt b) eine Glasübergangstemperatur im Bereich von -20 °C bis 30 °C aufweist.

**8.** Ein dispergierbares Vliesstoffsubstrat, das Folgendes beinhaltet:

a) ein Fasersubstrat; und
b) ein Vliesstoffbindemittel, das ein Emulsionspolymer beinhaltet, das einen Polydispersitätsindex von mindestens 10, ein Molekulargewicht im Gewichtsmittel von weniger als 1 000 000 und zu 8 Gewichtsprozent bis 22 Gewichtsprozent Säuremonomer aufweist;
c) eine Lotion mit einem pH-Wert im Bereich von 4 bis 5,5, die auch einen Puffer beinhaltet, der eine Mischung von Zitronensäure und Natriumcitrat beinhaltet.

**9.** Ein dispergierbares Tuchprodukt, hergestellt durch das Verfahren gemäß Anspruch 1.

**10.** Ein Babywischtuch, hergestellt mit dem dispergierbaren Tuchprodukt gemäß Anspruch 9.

**11.** Ein Körperpflegetuch, hergestellt mit dem dispergierbaren Tuchprodukt gemäß Anspruch 9.

**12.** Ein Reinigungstuch, hergestellt mit dem dispergierbaren Tuchprodukt gemäß Anspruch 9.

**13.** Ein Desinfektionstuch, hergestellt mit dem dispergierbaren Tuchprodukt gemäß Anspruch 9.

**14.** Ein Industrietuch, hergestellt mit dem dispergierbaren Tuchprodukt gemäß Anspruch 9.

**Revendications**

**1.** Un procédé pour former un substrat non tissé dispersible comprenant les étapes consistant à :

a) polymériser en émulsion au moins un monomère mono-éthyléniquement insaturé afin de former un polymère en émulsion ; et
b) neutraliser le polymère en émulsion avec un agent neutralisant sélectionné dans le groupe constitué d'une amine ayant un pKb de 4 à 7, d'un hydroxyde alcalin, et de combinaisons de ceux-ci afin de former le liant non tissé aqueux ;
c) mettre en contact un substrat non tissé avec ledit liant non tissé aqueux afin de former un substrat non tissé mis en contact ;
d) chauffer le substrat non tissé mis en contact jusqu'à une température allant de 120 °C à 220 °C afin de former un substrat non tissé mis en contact chauffé ;
e) immerger le substrat non tissé chauffé mis en contact dans un milieu aqueux ayant un pH final inférieur à 5,5 afin d'obtenir un substrat non tissé dispersible dans lequel le milieu aqueux contient un tampon comprenant un mélange d'acide citrique et de citrate de sodium.

**2.** Un procédé conformément à la revendication 1 dans lequel le polymère en émulsion comprend de l'acrylate d'éthyle, du styrène, de l'acide acrylique, et de l'acide itaconique.

**3.** Un procédé conformément à la revendication 1 dans lequel le polymère en émulsion a une masse moléculaire moyenne en poids inférieure à 1 000 000.

**4.** Un procédé conformément à la revendication 1 dans lequel le polymère en émulsion a un indice de polydispersité d'au moins 10.

**5.** Un procédé conformément à la revendication 1 dans lequel le substrat non tissé mis en contact chauffé est neutralisé avec une composition comprenant un acide avant d'être immergé dans le milieu aqueux dans l'étape e).

**6.** Un procédé conformément à la revendication 1 dans lequel l'étape de neutralisation aboutit à un polymère qui est neutralisé entre 80 % et 100 %.

**7.** Un procédé conformément à la revendication 1 dans lequel le polymère en émulsion, avant la neutralisation dans l'étape b), a une température de transition vitreuse dans la gamme allant de -20 °C à 30 °C.

**8.** Un substrat non tissé dispersible comprenant :

a) un substrat fibreux ; et

b) un liant non tissé comprenant un polymère en émulsion ayant un indice de polydispersité d'au moins 10, une masse moléculaire moyenne en poids inférieure à 1 000 000 et de 8 pour cent en poids à 22 pour cent en poids de monomère acide ;

c) une lotion ayant un pH dans la gamme allant de 4 à 5,5, qui comprend également un tampon comprenant un mélange d'acide citrique et de citrate de sodium.

**9.** Un produit de lingette dispersible préparé par le procédé de la revendication 1.

**10.** Une lingette pour bébé préparée avec le produit de lingette dispersible de la revendication 9.

**11.** Une lingette de soins personnels préparée avec le produit de lingette dispersible de la revendication 9.

**12.** Une lingette nettoyante préparée avec le produit de lingette dispersible de la revendication 9.

**13.** Une lingette désinfectante préparée avec le produit de lingette dispersible de la revendication 9.

**14.** Une lingette industrielle préparée avec le produit de lingette dispersible de la revendication 9.

**EP 3 240 923 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013154879 A
- US 4325856 A
- US 4654397 A
- US 4814373 A

**Non-patent literature cited in the description**

- **T.G. FOX.** *Bull. Am. Physics Soc.,* 1956, vol. 1 (3), 123
- Polymer Handbook. Interscience Publishers